# EUROPÄISCHE PATENTSCHRIFT

(11) **EP 2 344 353 B1**
(45) Veröffentlichungstag und Bekanntmachung des Hinweises auf die Patenterteilung: **20.08.2014**
(21) Anmeldenummer: 09771290.5
(22) Anmeldetag: 05.11.2009
(51) Int. Cl.: A61L 9/20, F24F 3/16, B60H 3/06, B60H 3/00

(54) **LUFTREINIGUNGSSYSTEM FÜR FAHRZEUGE**
AIR CLEANING SYSTEM FOR VEHICLES
SYSTÈME DE FILTRATION D'AIR POUR VÉHICULE

(30) Priorität: 05.11.2008 DE 102008055988
(43) Veröffentlichungstag der Anmeldung: 20.07.2011
(73) Patentinhaber: Johnson Controls GmbH, 51399 Burscheid (DE)
(72) Erfinder: GROSS, Bernd, 40764 Langenfeld (DE); WERNER, Hans-Georg, 40764 Langenfeld (DE); HESTERBERG, Joshua, 40822 Mettmann (DE); LEONG MAN LOONG, 25200 Kuantan, Pahang (MY)
(74) Vertreter: Schwöbel, Thilo K.
(86) Internationale Anmeldenummer: PCT/EP2009/007939
(87) Internationale Veröffentlichungsnummer: WO 2010/052001

(56) Entgegenhaltungen:
- WO-A1-02/078754
- DE-A1- 19 732 304
- DE-A1- 19 906 113
- JP-A- 2002 036 971
- US-A- 5 919 422

## Beschreibung

Die vorliegende Erfindung betrifft ein Luftreinigungssystem, insbesondere für Fahrzeuge oder Flugzeuge oder sonstige Verkehrsmittel.

Die US 5 919 422 zeigt eine Luftbehandlungsvorrichtung gemäß Oberbegriff des Anpruchs 1.

Insbesondere für den Fahrzeuginnenraum ist die Erzeugung reiner Luft, insbesondere im Wesentlichen keimfreier und keine organische Verschmutzungen enthaltender Luft, von hoher Wichtigkeit, insbesondere für das Wohlbefinden der Fahrzeuginsassen und damit auch für die Gewährleistung eines hohen Sicherheitsniveaus des Fahrzeugs. Es besteht daher ein großes wirtschaftliches Interesse, um die Fahrzeuginnenluft kostengünstig und effektiv sowie insbesondere mit einer kostengünstig herstellbaren, mit vergleichsweise geringem Energieverbrauch betreibbaren und bauraumkompakten Vorrichtung zu reinigen. Bekannte Vorrichtungen zur Reinigung von Luft betreffen Ozongeneratoren, Filtersysteme, Luftionisatoren und dergleichen. Filtersysteme eignen sich grundsätzlich nur zur Abscheidung von partikelförmigen gröberen Luftbestandteilen (Staub, Pollen, Mikroorganismen). Sie haben den Nachteil, dass die Filter regelmäßig ausgetauscht bzw. regeneriert werden müssen Bei der katalytischen Luftreinigung mittels Titandioxid werden durch eine photochemische Reaktion Hydroxyl Radikale OH- und Super-Oxide O₂⁻ gebildet. Damit werden alle organischen Spurenstoffmoleküle bzw. Luftschadstoffmoleküle in Gegenwart von Luftsauerstoff vollständig oxidiert, d.h. es findet eine quasi kalte Verbrennungsreaktion von Luftsauerstoff unter Bildung von Kohlendioxid (CO₂) und Wasser (H₂O) und ggfs. weiteren ungiftigen Produkten statt. Gleichzeitig erfolgt eine Zersetzung/Umwandlung der Schadstoffe.

Nachteile bei bekannten Verfahren zur photokatalytischen Luftreinigung bestehen in ihrer geringen Effektivität, insbesondere aufgrund einer geringen Reaktionsfläche im Reaktionsraum, einer dadurch notwendigen höheren Verweilzeit im Reaktionsraum sowie einer unvollständigeren Reaktion und dem Austragen (eines größeren Teils) von Primärprodukten mit dem Luftstrom.

Der Erfindung lag deshalb die Aufgabe zugrunde, ein Luftreinigungssystem für Fahrzeuge bereitzustellen, mittels dem trotz kleinstem Bauraum eine maximale Luftreinigungsleistung möglich ist. Insbesondere soll es erfindungsgemäß ermöglicht werden, dass das Luftreinigungssystem bzw. eine Luftreinigungsvorrichtung derart bauraumkompakt realisierbar ist, dass ein Einbau in einen Fahrzeugsitz möglich ist. Gelöst wird die Aufgabe mittels eines Luftreinigungssystems gemäß Anspruch 1 wobei das Reaktionsflächenelement bevorzugt wenigstens 60%, besonders bevorzugt wenigstens 70%, der Innenoberfläche der Luftreinigungsvorrichtung einnimmt.

Es ist hierdurch erfindungsgemäß vorteilhaft möglich, eine Luftreinigungsvorrichtung zu realisieren, bei der auf kleinem Bauraum und insbesondere durch eine besondere Bauform eines Luftkanals eine maximale Reaktionsoberfläche sowie durch eine besondere Anordnung einer oder mehrerer Lichtquellen und/oder durch eine besondere Anzahl von Lichtquellen eine hocheffektive, insbesondere photokatalytische Reinigungsleistung ermöglicht wird.

Mit dem erfindungsgemäßen Luftreinigungssystem bzw. der erfindungsgemäßen Luftreinigungsvorrichtung ist es erfindungsgemäß vorteilhaft möglich, dass die Fahrzeuginnenraumluft innerhalb vergleichsweise kurzer Zeit, beispielsweise innerhalb von 30 Minuten, zu einem hohen Anteil gereinigt wird, insbesondere von unangenehmen Gerüchen gereinigt wird, und dass Keime bzw. Bakterien bzw. Viren wirksam abgetötet werden. Ferner ist es erfindungsgemäß vorgesehen, dass gereinigte Luft bevorzugt an solche Stellen des Fahrzeuginnenraums zuerst gelangt, an denen die Wahrscheinlichkeit, dass ein Passagier die Luft einatmet, vergleichsweise groß ist. Solche Stellen des Fahrzeuginnenraums betreffen beispielsweise im wesentlichen der gesamte Bereich unterhalb des Dachhimmels des Fahrzeugs, weil sich in diesem räumlichen Bereich bevorzugt die Köpfe der Fahrzeuginsassen befinden.

Erfindungsgemäß ist das Reaktionsflächenelement im Wesentlichen im gesamten Luftführungsvolumen der Luftreinigungsvorrichtung angeordnet; beispielsweise füllt das Reaktionsflächenelement das Luftführungsvolumen im Wesentlichen vollständig aus (etwa in Form eines Füllmaterials, welches oberflächenbeschichtet ist) bzw. erstreckt sich zumindest über den gesamten Bereich des Luftführungsvolumens (etwa in Form eines Gitters oder Netzes oder Drahtgeflechts oder eines oberflächenbeschichteten Faserelements, welches sich über den gesamten Bereich des Luftführungsvolumens erstreckt). Erfindungsgemäß ist es in diesem Zusammenhang besonders bevorzugt, wenn das Reaktionsflächenelement in wenigstens 60% des Luftführungsvolumens angeordnet ist, bevorzugt in wenigstens 80% des Luftführungsvolumens oder auch in wenigstens 90% des Luftführungsvolumens.

Erfindungsgemäß ist es bevorzugt vorgesehen, dass das Katalysatormaterial Titandioxid enthält. Hierdurch kann eine besonders gute Reinigungswirkung erzielt werden. Die Reinigungswirkung kann ferner dadurch erhöht werden, dass die Luftreinigungsvorrichtung ein weiteres Luftführungsvolumen (getrennt bzw. vorgeschaltet vom Luftführungsvolumen) aufweist, wobei das weitere Luftführungsvolumen ein Zeolith-Material und/oder ein Silber-Substrat-Material als ein weiteres Katalysatormaterial aufweist, d.h. ohne Anwesenheit von Titandioxid als Katalysatormaterial und auch ohne Beleuchtung mit einer UV-A-Lichtquelle im weiteren Luftführungsvolumen.

Erfindungsgemäß ist es weiterhin dass das Reaktionsflächenelement ein oberflächenbeschichtetes Trägerelement, ein Drahtgeflecht oder bevorzugt ein Fasermaterial aufweist, bevorzugt ein Zeolith-Material und/oder ein Silber-Substrat-Material. Hierdurch kann eine besonders gut definierte räumliche Anordnung des Materials der Oberflächenbeschichtung, erfindungsgemäß insbesondere ein Titandioxid enthaltendes Material, gewährleistet werden, die auch über die Lebensdauer der Luftreinigungsvorrichtung im Wesentlichen unverändert bleibt. Hierdurch kann auch eine besonders gut definierte und effiziente Beleuchtung der Oberflächenbeschichtung durch die Lichtquelle bzw. die Lichtquellen bewerkstelligt werden. Ferner ist es hierdurch erfindungsgemäß auch möglich, den Strömungswiderstand der durch die Luftreinigungsvorrichtung hindurchströmenden Luft vergleichsweise gering zu halten. Durch die definierte Anordnung des oberflächenbeschichteten Trägerelements ist es ferner auch vorteilhaft möglich, dass der Strömungswiderstand über die Gebrauchsdauer der Luftreinigungsvorrichtung im Wesentlichen gleich bleibt bzw. sich in vergleichsweise gut definierter Weise ändert. Weiterhin ist es ebenfalls erfindungsgemäß dass das Reaktionsflächenelement als oberflächenbeschichtetes Schüttgut ausgebildet ist, in Form von Glasrohrabschnitten und/oder in Form von Glaskugeln. Hierbei kann in einfacher Weise eine besonders große Oberfläche der oberflächenwirksamen Substanz, d.h. der Oberflächenbeschichtung, erzielt werden. Ferner ist es hierdurch erfindungsgemäß auch möglich, den Strömungswiderstand der durch die Luftreinigungsvorrichtung hindurchströmenden Luft vergleichsweise gering zu halten.

Erfindungsgemäß ist es ferner bevorzugt, dass die Luftreinigungsvorrichtung ein Basiselement und ein Deckelelement aufweist, wobei das Reaktionsflächenelement mit dem Basiselement oder dem Deckelement einstückig verbunden ist, insbesondere in Form von in das Luftführungsvolumen hineinragenden oberflächenbeschichteten stabförmigen Elementen. Hierdurch kann ebenfalls eine besonders gut definierte räumliche Anordnung des Materials der Oberflächenbeschichtung gewährleistet werden, die auch über die Lebensdauer der Luftreinigungsvorrichtung im Wesentlichen unverändert bleibt. Die Beleuchtung der Oberflächenbeschichtung kann damit besonders gut definiert und effizient gestaltet werden. Ferner ist es hierdurch erfindungsgemäß auch möglich, den Strömungswiderstand der durch die Luftreinigungsvorrichtung hindurchströmenden Luft vergleichsweise gering zu halten. Durch die definierte Anordnung der in das Luftführungsvolumen hineinragenden oberflächenbeschichteten stabförmigen Elemente ist es ferner auch vorteilhaft möglich, dass der Strömungswiderstand über die Gebrauchsdauer der Luftreinigungsvorrichtung im Wesentlichen gleich bleibt bzw. sich in vergleichsweise gut definierter Weise ändert.

Erfindungsgemäß ist bevorzugt, dass das Luftführungsvolumen eine wenigstens zweifache Biegung um mehr als 90° zwischen Lufteinlass und Luftauslass aufweist bzw. dass das Luftführungsvolumen zwischen Lufteinlass und Luftauslass spiralförmig ausgebildet ist. Hierdurch kann auf kleinem Raum eine besonders gute Luftreinigung erzielt werden.

Besonders bevorzugt ist es, wenn die Luftreinigungsvorrichtung in eine Fahrzeugkarosserie, insbesondere den Dachbereich der Fahrzeugkarosserie, derart integriert ist, dass ein Teil der Fahrzeugkarosserie als oberflächenbeschichtete Wandung des Luftführungsvolumens vorgesehen ist. Hierdurch kann in vorteilhafter Weise beispielsweise der Raum zwischen einem Dachhimmel und der Fahrzeugkarosserie effizient genutzt werden.

Weiterhin ist es bevorzugt, dass die Luftreinigungsvorrichtung in einer Fahrzeugkomponente, insbesondere einem Dachhimmel, derart integriert ist, dass ein Teil der Fahrzeugkomponente als oberflächenbeschichtete Wandung des Luftführungsvolumens vorgesehen ist. Hierdurch kann ebenfalls der Raum zwischen einem Dachhimmel und der Fahrzeugkarosserie effizient genutzt werden, wobei jedoch keine Veränderungen an der Karosserie erforderlich sind.

Ein weiterer Gegenstand der vorliegenden Erfindung betrifft eine Fahrzeugkomponente, insbesondere eine Innenausstattungskomponente, mit einer integrierten erfindungsgemäßen Luftreinigungsvorrichtung, wobei die Fahrzeugkomponente ein Dachhimmel oder eine Hutablage oder eine Mittelkonsole oder eine Türverkleidung oder A-Säulenverkleidung oder eine B-Säulenverkleidung oder eine C-Säulenverkleidung oder eine Instrumententafel ist.

Ferner bezieht sich die Erfindung auch auf die Verwendung einer Fahrzeugkarosserie oder eines Teils davon oder einer Fahrzeugkomponente oder eines Teils davon als Wandung einer erfindungsgemäßen Luftreinigungsvorrichtung.

Ausführungsbeispiele der Erfindung sind in der Zeichnung dargestellt.

Die Figuren 1 bis 12 zeigen zehn unterschiedliche erfindungsgemäße Ausführungsbeispiele des erfindungsgemäßen Luftreinigungssystems bzw. der erfindungsgemäßen Luftreinigungsvorrichtung.
In Figur 1 ist ein erstes Ausführungsbeispiel des erfindungsgemäßen Luftreinigungssystems 10 bzw. der erfindungsgemäßen Luftreinigungsvorrichtung 10 dargestellt.
In Figur 2 ist ein zweites Ausführungsbeispiel des erfindungsgemäßen Luftreinigungssystems 10 bzw. der erfindungsgemäßen Luftreinigungsvorrichtung 10 dargestellt.
In Figur 3 ist ein drittes Ausführungsbeispiel des erfindungsgemäßen Luftreinigungssystems 10 bzw. der erfindungsgemäßen Luftreinigungsvorrichtung 10 dargestellt.
In Figur 4 ist ein viertes Ausführungsbeispiel des erfindungsgemäßen Luftreinigungssystems 10 bzw. der erfindungsgemäßen Luftreinigungsvorrichtung 10 dargestellt.
In Figur 5 ist ein fünftes Ausführungsbeispiel des erfindungsgemäßen Luftreinigungssystems 10 bzw. der erfindungsgemäßen Luftreinigungsvorrichtung 10 dargestellt.
In den Figuren 6 und 7 ist ein sechstes Ausführungsbeispiel des erfindungsgemäßen Luftreinigungssystems 10 bzw. der erfindungsgemäßen Luftreinigungsvorrichtung 10 dargestellt.
In den Figuren 8 und 9 ist ein siebtes Ausführungsbeispiel des erfindungsgemäßen Luftreinigungssystems 10 bzw. der erfindungsgemäßen Luftreinigungsvorrichtung 10 dargestellt.
In Figur 10 ist ein achtes Ausführungsbeispiel des erfindungsgemäßen Luftreinigungssystems 10 bzw. der erfindungsgemäßen Luftreinigungsvorrichtung 10 dargestellt.
In Figur 11 ist ein neuntes Ausführungsbeispiel des erfindungsgemäßen Luftreinigungssystems 10 bzw. der erfindungsgemäßen Luftreinigungsvorrichtung 10 dargestellt.
In Figur 12 ist ein zehntes Ausführungsbeispiel des erfindungsgemäßen Luftreinigungssystems 10 bzw. der erfindungsgemäßen Luftreinigungsvorrichtung 10 dargestellt.

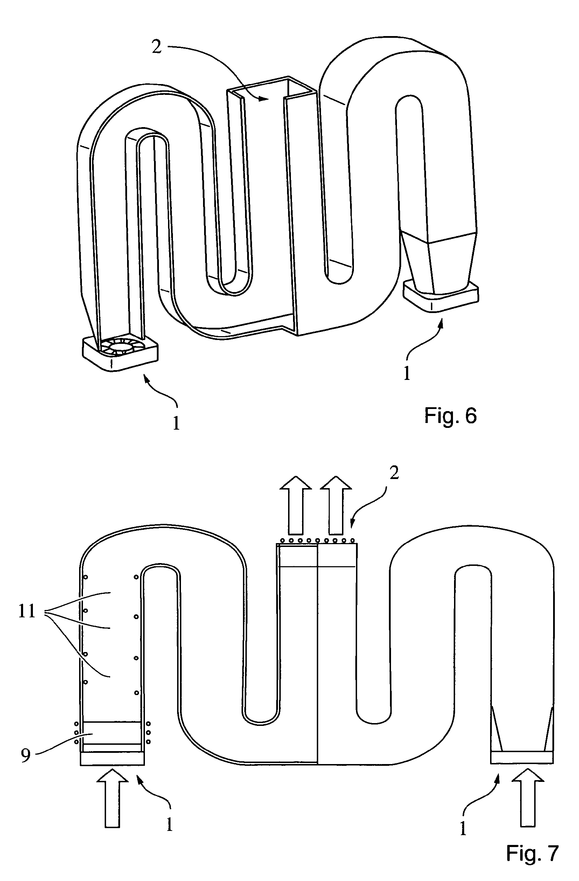

In allen Ausführungsbeispielen umfasst die Luftreinigungsvorrichtung 10 bzw. das Luftreinigungssystem 10 einen Lufteinlass 1 und einen Luftauslass 2. Ferner weist die Luftreinigungsvorrichtung 10 bzw. das Luftreinigungssystem 10 ein Gehäuse 5, eine Lichtquelle 4 oder eine Mehrzahl von Lichtquellen 4 und ein Reaktionsoberflächenelement 3 oder eine Mehrzahl von Reaktionsoberflächenelementen 3 auf. Im ersten bis fünften Ausführungsbeispiel ist als Reaktionsoberflächenelement 3 ein mit einem Katalysatormaterial (als Oberflächenbeschichtung) versehenes Gitter oder Drahtgeflecht vorgesehen. Das Katalysatormaterial ist insbesondere als ein Titandioxidmaterial bzw. Titandioxid umfassend ausgebildet, beispielsweise als ein nanostrukturiertes Katalysatormaterial (Nano-Titandioxid). Im ersten bis zehnten Ausführungsbeispiel ist als Lichtquelle 4 eine Ultraviolettstrahlung (UV-Strahlung) erzeugende Lichtquelle 4 vorgesehen, insbesondere eine UV-A-Strahlung erzeugende Lichtquelle 4, insbesondere in Form einer UV-A-Röhre und/oder in Form einer UV-A-Leuchtdiode oder mehrerer UV-A-Leuchtdioden. Die Verwendung einer oder mehrerer UV-A-Röhre(n) ist insbesondere bei der ersten, zweiten und fünften Ausführungsbeispiel besonders bevorzugt. Die Verwendung einer oder mehrerer UV-A-Leuchtdiode(n) ist insbesondere bei der dritten, vierten und sechsten bis zehnten Ausführungsbeispiel besonders bevorzugt. Bei der Verwendung von UV-A-Leuchtdioden als Lichtquelle ist es erfindungsgemäß besonders bevorzugt, dass UV-A-Lichtleiter-Folien zur Verteilung des Lichts Verwendung finden. In allen Ausführungsbeispielen ist (pro separiertem Luftführungsvolumen bzw. pro separierten Luftführungsvolumen und weiterem Luftführungsvolumen) wenigstens ein Lüfter, beispielsweise ein Axial-Lüfter, vorgesehen, der die verunreinigte Luft durch das Gehäuse drückt oder zieht. Der Lüfter kann entweder (in Strömungsrichtung der Luft) vor dem Gehäuse 5 angeordnet sein oder aber (in Strömungsrichtung der Luft) nach dem Gehäuse 5 angeordnet sein. Die durch die Luftreinigungsvorrichtung hindurchströmende Luft kommt dabei in Kontakt mit dem Reaktionsflächenelement 3 bzw. mit dessen beschichteter Oberfläche und wird gereinigt, insbesondere aufgrund der Beleuchtungswirkung der Oberfläche der Reaktionsflächenelemente 3 durch die Lichtquelle 4 bzw. die Lichtquellen 4. Eine besonders hohe Effizienz der Luftreinigung wird hierbei durch die große Oberfläche des Reaktionsflächenelements 3 und die großflächige Lichtquelle 4 bzw. die große Anzahl von Lichtquellen 4 realisiert. Aufgrund der photokatalytischen Wirkung von UV-A-Licht und Titandioxid, insbesondere Nano-Titandioxid) werden Luftschadstoffe (beispielsweise Rauch/Gerüche/Keime/Bakterien) chemisch umgewandelt bzw. eliminiert. Die Effizienz der Luftreinigung kann erfindungsgemäß ferner dadurch erhöht werden, dass Nano-Zeolith-Substrate und/oder Nano-Silber-Substrate als Reaktionsflächenelement verwendet werden. Alternativ zu einer Verwendung eines Zeolith-Materials und/oder eines Silber-Substrat-Materials als Reaktionsflächenelement kann es erfindungsgemäß vorgesehen sein, dass diese Materialien in einem weitere (vom Luftführungsvolumen getrennten) Luftführungsvolumen als ein weiteres Katalysatormaterial 9 vorkommen, wobei ein solches weiteres Luftführungsvolumen vorzugsweise im Bereich des Einlasses 1 bzw. im Bereich des Auslasses 2 angeordnet ist. Diese Ausführungsvariante ist lediglich in der Figur 7 dargestellt.

Die Formgebung des Reaktionsflächenelements 3, der Lichtquelle 4 und des Gehäuses 5 unterscheidet sich bei den Ausführungsbeispielen.

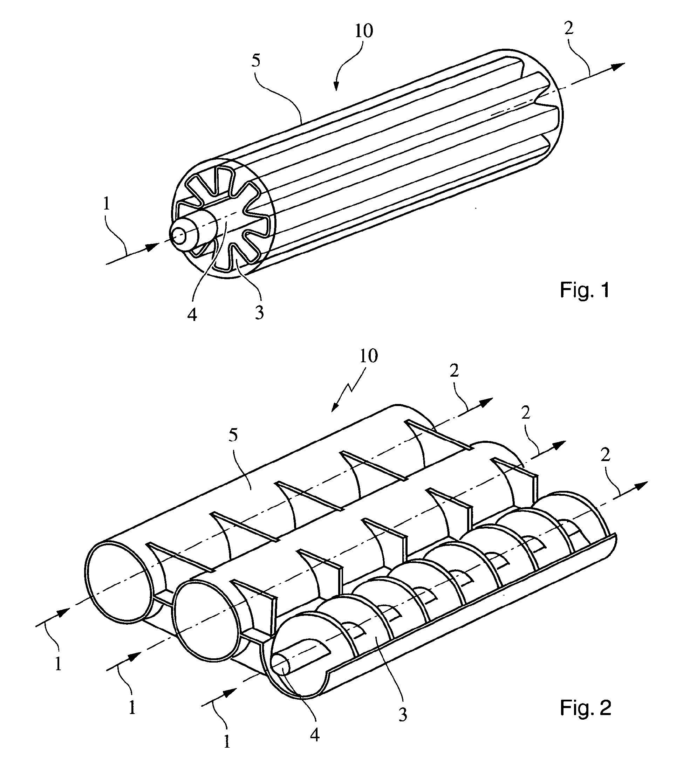

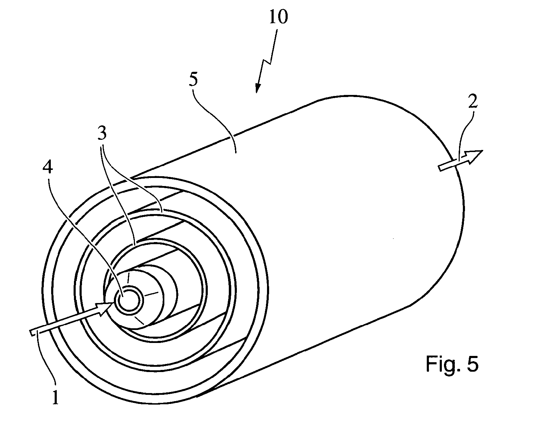

So ist das Gehäuse 5 beim ersten, zweiten und fünften Ausführungsbeispiel (Figuren 2, 3 und 5) zumindest abschnittsweise rohrförmig bzw. zylinderförmig ausgebildet. Der Lufteinlass 1 und der Luftauslass 2 sind an den Stirnseiten des Rohres vorgesehen. Im Zentrum des Rohres ist die Lichtquelle 4 angeordnet, insbesondere als UV-A-Röhre, wobei jedoch auch ein Lichtleitersystem mit einer gemeinsamen, zentralen UV-A-Lichtquelle 4 vorgesehen sein kann. Im Bereich radial zwischen der Lichtquelle 4 und dem Gehäuse 5 ist das Reaktionsflächenelement 3 angeordnet. Auf der Lufteinlassseite ist ein Lüfter (nicht dargestellt), insbesondere ein Axial-Lüfter, angeordnet. Die gereinigte Luft tritt auf der Luftauslassseite aus. Beim ersten Ausführungsbeispiel (Figur 1) ist das Reaktionsflächenelement 3 im Querschnitt insbesondere sternförmig bzw. mäanderförmig gestaltet. Beim zweiten Ausführungsbeispiel (Figur 2) ist das Reaktionsflächenelement 3 beispielsweise spiralförmig ausgebildet. Beim fünften Ausführungsbeispiel (Figur 5) ist das Reaktionsflächenelement 3 im Wesentlichen rohrförmig gestaltet, wobei zwischen der Lichtquelle 4 und dem Gehäuse beispielsweise zwei Reaktionsflächenelemente 3 unterschiedlichen Durchmessers vorgesehen sind.

Eine Mehrzahl von rohrförmigen oder zylindrischen Abschnitten können beim ersten, zweiten und fünften Ausführungsbeispiel (Figuren 1, 2 und 5) auch parallel und/oder hintereinandergeschaltet vorgesehen sein. Eine Parallelschaltung ist beispielhaft in Figur 2 dargestellt. Eine Hintereinanderschaltung zweier rohrförmiger oder zylindrischer Abschnitte kann beispielsweise dadurch erfolgen, dass die Luftauslassseite des einen Rohres mit der Lufteinlassseite des anderen Rohres gekoppelt ist und die Luft zunächst das eine Rohrdurchströmt, anschließend umgelenkt wird und anschließend das andere Rohr (insbesondere in Gegenrichtung) durchströmt. Mit fünf solcher rohrförmiger oder zylindrischer Abschnitte kann beispielsweise jeweils ein Lüfter an den beiden äußeren Luftkanälen vorgesehen sein, wobei die inneren Luftkanäle so miteinander gekoppelt sind, dass ein zentraler Auslass in der Mitte entsteht.

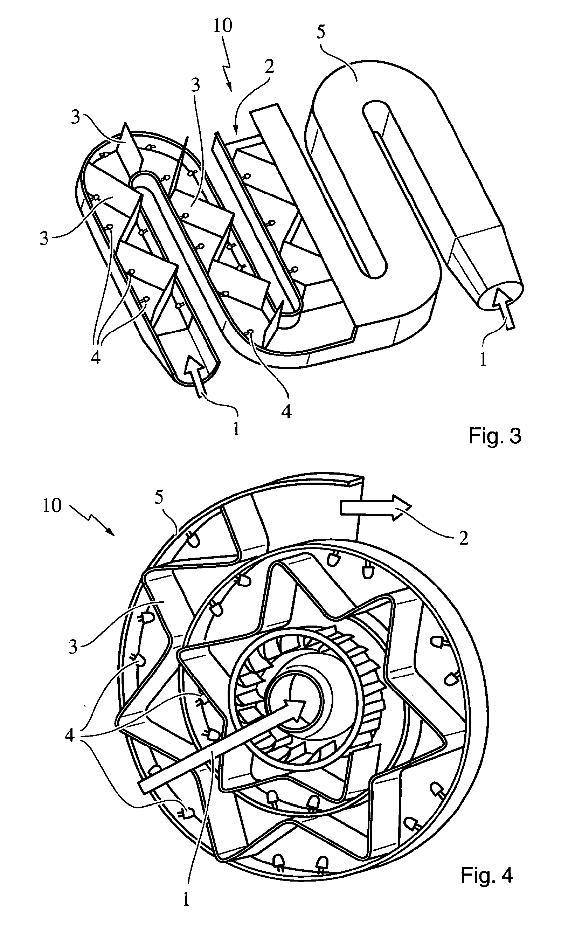

Beim dritten und vierten Ausführungsbeispiel (Figuren 3 und 4) ist das Reaktionsflächenelement 3 hauptsächlich zickzackförmig bzw. mäandrierend bzw. ziehharmonikaähnlich über den Querschnitt des Luftkanals des Gehäuses 5 vorgesehen. Der Luftkanal des Gehäuses ist beim dritten Ausführungsbeispiel (Figur 3) selbst mäandrierend mit einem ersten Schenkel, einer Umkehrung (des Luftführungsvolumens um etwa 180°), einem zweiten Schenkel, einer weiteren Umkehrung (des Luftführungsvolumens um etwa 180°) und einem dritten Schenkel vorgesehen. Der Luftkanal des Gehäuses ist beim vierten Ausführungsbeispiel (Figur 4) schneckenförmig bzw. spiralförmig vorgesehen. Im Zentrum der Spirale, welche den Lufteinlass 1 darstellt, ist insbesondere ein Radial-Lüfter vorgesehen.

Beim sechsten und siebten Ausführungsbeispiel (Figuren 6 bis 9) ist der Luftkanal des Gehäuses wiederum selbst mäandrierend mit einem ersten Schenkel, einer Umkehrung (des Luftführungsvolumens um etwa 180°), einem zweiten Schenkel, einer weiteren Umkehrung (des Luftführungsvolumens um etwa 180°) und einem dritten Schenkel vorgesehen.

Als Reaktionsflächenelement 3 ist beim sechsten Ausführungsbeispiel (Figuren 6 und 7) ein Schüttgut 11 vorgesehen (bzw. es ist eine Vielzahl von Reaktionsflächenelementen 3) vorgesehen. Beispielsweise ist als Reaktionsflächenelemente 3 eine Mehrzahl von kurzen Glasröhrchen (bzw. Glasrohrabschnitten) vorgesehen, die oberflächenbeschichtet vorgesehen und über im Wesentlichen den gesamten Raum des Luftführungsvolumens verteilt sind. Dies ist in der Figur 7 angedeutet. Alternativ zu der Verwendung von Glasröhrchen ist es möglich, oberflächenbeschichtete Glaskugeln zu verwenden. Ferner ist es erfindungsgemäß auch möglich, eine Mischung aus oberflächenbeschichteten Glasröhrchen und Glaskugeln zu verwenden. Die Reaktionsflächenelemente 3 sind in allen Fällen des sechsten Ausführungsbeispiels (Figuren 6 und 7) über im Wesentlichen den gesamten Raum des Luftführungsvolumens verteilt vorgesehen. Alternativ oder kumulativ zu oberflächenbeschichteten Glaselementen (Glasrohrabschnitte oder Glaskugeln) können auch transparente oder intransparente Kunststoffrohrabschnitte (Kunststoffröhrchen) oder kugelförmige transparente oder intransparente sonstige Kunststoffstrukturen als Reaktionsflächenelemente 3 Verwendung finden.

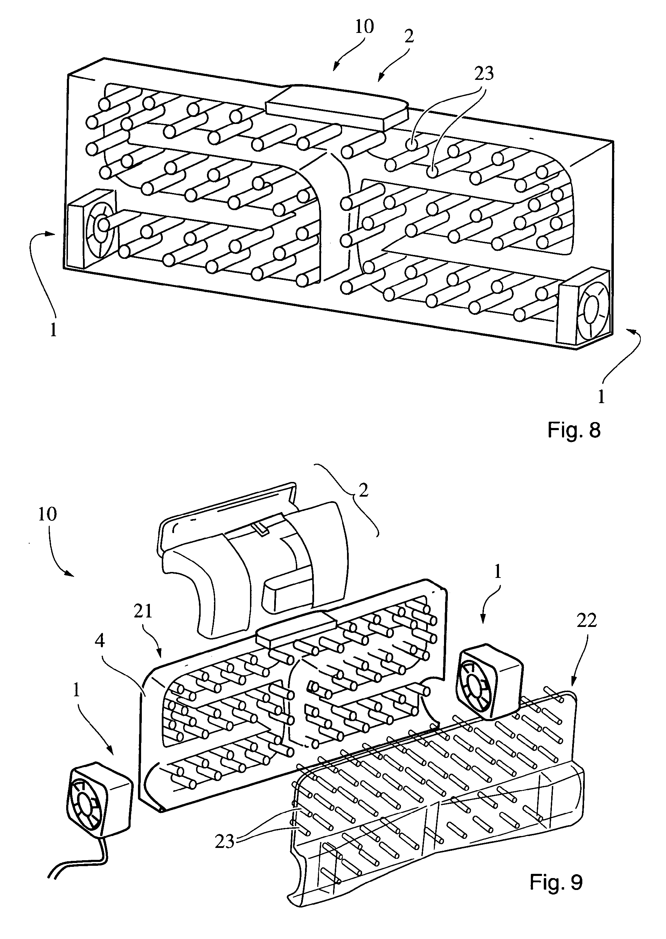

Als Reaktionsflächenelement 3 ist beim siebten Ausführungsbeispiel (Figuren 8 und 9) vorgesehen, dass die Luftreinigungsvorrichtung 10 ein Basiselement 21 und ein Deckelelement 22 aufweist, wobei das Reaktionsflächenelement 3 mit dem Basiselement 21 oder dem Deckelement 22 einstückig verbunden ist, insbesondere in Form von in das Luftführungsvolumen hineinragenden oberflächenbeschichteten stabförmigen Elementen 23. Es kann selbstverständlich erfindungsgemäß auch vorgesehen sein, dass stabförmige Elemente 23 sowohl vom Basiselement 21 als auch vom Deckelement 22 ausgehend ausgebildet sind. Ferner ist es erfindungsgemäß beim siebten Ausführungsbeispiel (Figuren 8 und 9) vorteilhaft möglich, dass quasi die gesamte innere Oberfläche der Luftreinigungsvorrichtung 10 oberflächenbeschichtet ist und damit als Reaktionsflächenelement 3 wirkt, wobei jedoch ausreichend Platz vorgesehen sein muss, um die Lichtquelle 4 bzw. die Mehrzahl von Lichtquellen 4 anzuordnen.

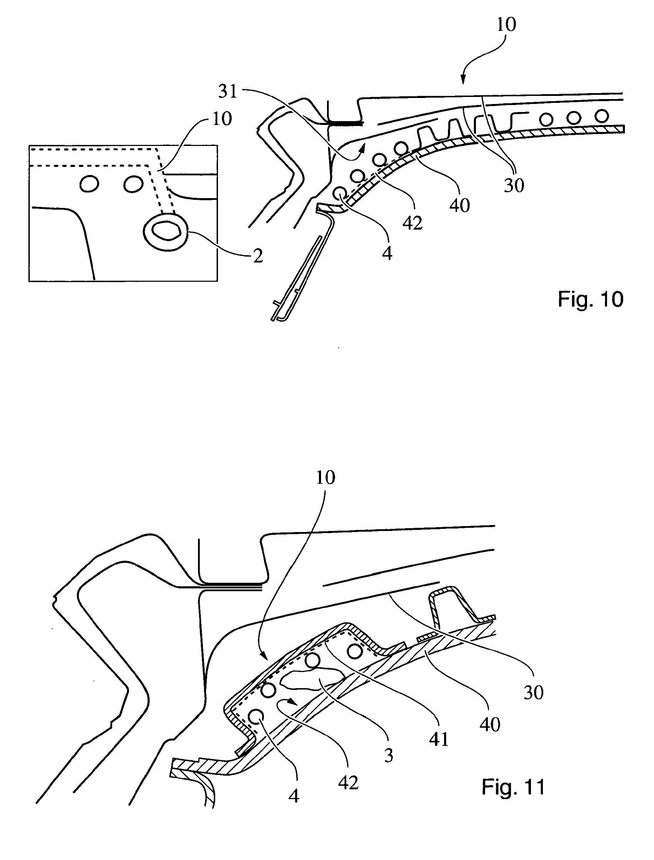

Gemäß dem achten Ausführungsbeispiel (Figur 10) ist es vorgesehen, dass die Luftreinigungsvorrichtung 10 in eine Fahrzeugkarosserie 30, insbesondere den Dachbereich der Fahrzeugkarosserie 30, derart integriert ist, dass ein Teil der Fahrzeugkarosserie 30 als oberflächenbeschichtete Wandung 31 des Luftführungsvolumens vorgesehen ist. Dies ist in der Figur 10 am Beispiel eines Dachbereichs der Fahrzeugkarosserie und eines dem Innenraum des Fahrzeugs zugewandten Dachhimmels als Beispiel einer Fahrzeugkomponente 40 schematisch dargestellt. Im linken Teil der Figur 10 ist eine Ansicht des Dachhimmels dargestellt, wie sie einem Insassen des Fahrzeugs erscheinen mag. Mit gestrichelter Linie ist ein Luftführungskanal bzw. eine Luftreinigungsvorrichtung 10 auf der dem Fahrzeuginsassen abgewandten Seiten des Dachhimmels 40 angedeutet, der in einen Luftauslass 2 mündet. Im rechten Teil der Figur 10 ist schematisch eine Schnittdarstellung durch einen solchen Luftführungskanal bzw. eine solche Luftreinigungsvorrichtung 10 dargestellt. Eine karosserieseitige Wandung 31 des Luftführungskanals bzw. der Luftreinigungsvorrichtung 10 ist oberflächenbeschichtet vorgesehen und trägt zur Luftreinigung bei durch den Luftkanal strömender Luft bei. Lichtquellen 4 sind auf der dem Fahrzeuginsassen abgewandten Seite der Fahrzeugkomponente 40 vorgesehen. Alternativ oder kumulativ kann es erfindungsgemäß beim achten Ausführungsbeispiel (Figur 10) auch vorgesehen sein, dass die der Karosserie zugewandte Seite 42 der Fahrzeugkomponente 40 zusätzlich oberflächenbeschichtet vorgesehen ist. Beim achten Ausführungsbeispiel (Figur 10) ist es besonders vorteilhaft, dass der Bauraum zwischen der Fahrzeugkomponente 40 (also beispielsweise einem Dachhimmel oder einer sonstigen Verkleidung innerhalb eines Fahrzeugs) einerseits und der Karosserie 30 andererseits besonders effizient zur Luftreinigung verwendet werden kann. Es kann alternativ oder kumulativ auch vorgesehen sein, dass die Fahrzeugkomponente 40 zumindest in Teilbereichen derart luftdurchlässig gestaltet wird, dass keine äußerlich erkennbaren Luftauslassöffnungen erkennbar sind, sondern durch das Material der Fahrzeugkomponente die Luft hindurchströmt. Wenn die der Karosserie 30 zugewandte Seite 42 der Fahrzeugkomponente 40 oberflächenbeschichtet ist, kann zusätzlich eine Luftreinigungswirkung erzielt werden.

Gemäß dem neunten Ausführungsbeispiel (Figur 11) ist es vorgesehen, dass die Luftreinigungsvorrichtung 10 in einer Fahrzeugkomponente 40, insbesondere einem Dachhimmel, derart integriert ist, dass ein Teil der Fahrzeugkomponente 40 als oberflächenbeschichtete Wandung 41 des Luftführungsvolumens vorgesehen ist. Im in der Figur 11 dargestellten Beispiel ist schematisch eine Schnittdarstellung durch einen solchen Luftführungskanal bzw. eine solche Luftreinigungsvorrichtung 10 dargestellt. Eine oberflächenbeschichtete Wandung 41 der Luftreinigungsvorrichtung 10 ist Teil der Fahrzeugkomponente 40 (d.h. beispielsweise eines Verkleidungselements wie beispielsweise des Dachhimmels) und trägt zur Luftreinigung bei durch den Luftkanal strömender Luft bei. Lichtquellen 4 sind auf der dem Fahrzeuginsassen abgewandten Seite der Fahrzeugkomponente 40 vorgesehen. Alternativ oder kumulativ kann es erfindungsgemäß beim neunten Ausführungsbeispiel (Figur 11) auch vorgesehen sein, dass die der oberflächenbeschichteten Wandung 41 zugewandte Seite 42 der Fahrzeugkomponente 40 zusätzlich oberflächenbeschichtet vorgesehen ist. Beim neunten Ausführungsbeispiel (Figur 11) ist es besonders vorteilhaft, dass der Bauraum zwischen der Fahrzeugkomponente 40 (also beispielsweise einem Dachhimmel oder einer sonstigen Verkleidung innerhalb eines Fahrzeugs) einerseits und der Karosserie 30 andererseits besonders effizient zur Luftreinigung verwendet werden kann. Es kann alternativ oder kumulativ auch vorgesehen sein, dass die Fahrzeugkomponente 40 zumindest in Teilbereichen derart luftdurchlässig gestaltet wird, dass keine äußerlich erkennbaren Lufauslassöffnungen erkennbar sind, sondern durch das Material der Fahrzeugkomponente die Luft hindurchströmt. Wenn die der oberflächenbeschichteten Wandung 41 zugewandte Seite 42 der Fahrzeugkomponente 40 oberflächenbeschichtet ist, kann zusätzlich eine Luftreinigungswirkung erzielt werden.

Falls beim achten und neunten Ausführungsbeispiel (Figur 11) lediglich eine Wandung oder mehrere Wandungen der Luftreinigungsvorrichtung 10 oberflächenbeschichtet sind, dann wird das Reaktionsflächenelement 3 durch diese Wandungen gebildet. Alternativ oder kumulativ zu einer solche Ausführung ist es beim achten und neunten Ausführungsbeispiel (Figuren 10 und 11) jedoch auch möglich, dass Reaktionsflächenelemente 3 entweder in Form von oberflächenbeschichteten Gittern oder Geweben oder aber in Form von oberflächenbeschichtetem Schüttgut in die Luftreinigungsvorrichtung 10 eingebracht wird, um so die Luftreinigungswirkung noch weiter zu steigern.

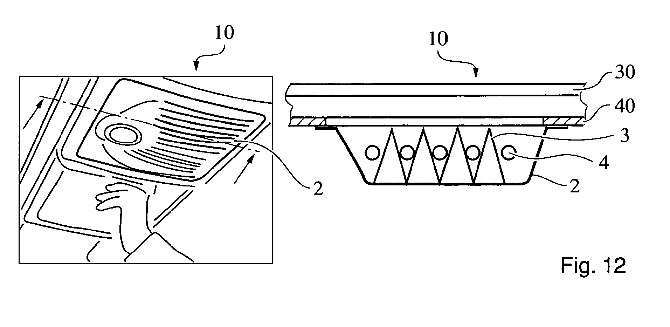

Gemäß dem zehnten Ausführungsbeispiel (Figur 12) ist es vorgesehen, dass eine erfindungsgemäße Luftreinigungsvorrichtung 10 gemäß dem ersten bis siebten Ausführungsbeispiel (Figuren 1 bis 9) in eine Fahrzeugkomponente 40 eingebaut wird, beispielsweise im Bereich des Dachhimmels. So ist beispielsweise in Figur 12 auf der linken Seite eine Ansicht einer solchen in den Dachhimmel integrierten Luftreinigungsvorrichtung 10 schematisch in einer perspektivischen Darstellung abgebildet, wobei ein Luftauslass 2 erkennbar ist. Auf der rechten Seite der Figur 12 ist in schematischer Weise ein Querschnitt der Luftreinigungsvorrichtung 10 dargestellt aus dem die Fahrzeugkomponente 40, die Karosserie 30, der Auslass 2, die Lichtquelle 4 und ein Reaktionsflächenelement 3 hervorgeht.

Alternativ zu einer Anordnung der Luftreinigungsvorrichtung 10 im Bereich des Dachhimmels kann es erfindungsgemäß auch vorteilhaft vorgesehen sein, die Luftreinigungsvorrichtung 10 im Bereich der Hutablage und/oder im Bereich einer Mittelkonsole des Fahrzeugs und/oder im Bereich einer Türverkleidung des Fahrzeugs und/oder im Bereich einer A-Säulenverkleidung des Fahrzeugs und/oder im Bereich eine B-Säulenverkleidung des Fahrzeugs und/oder im Bereich einer C-Säulenverkleidung des Fahrzeugs anzuordnen.

### Bezugszeichenliste

- 1: Lufteinlass
- 2: Luftauslass
- 3: Reaktionsflächenelement
- 4: Lichtquelle
- 5: Gehäuse
- 9: weiteres Katalysatormaterial
- 10: Luftreinigungssystem / Luftreinigungsvorrichtung
- 21: Basiselement
- 22: Deckelement
- 23: stabförmige Elemente
- 30: Fahrzeugkarosserie
- 31: oberflächenbeschichtete Wandung
- 40: Fahrzeugkomponente
- 41: oberflächenbeschichtete Wandung des Luftführungsvolumens
- 42: der Karosserie zugewandte Seite

## Patentansprüche

1. Luftreinigungsvorrichtung (10) für ein Fahrzeug, mit einem Lufteinlass (1), mit einem Luftauslass (2) und mit einem Luftführungsvolumen zwischen Lufteinlass (1) und Luftauslass (2), wobei in dem Luftführungsvolumen wenigstens ein Reaktionsflächenelement (3) vorgesehen ist, im Luftführungsvolumen wenigstens eine Lichtquelle (4) für ultraviolettes Licht angeordnet ist, dass die Oberfläche des Reaktionsflächenelements (3) ein Katalysatormaterial aufweist und dass das Reaktionsflächenelement (3) im wesentlichen im gesamten Luftführungsvolumen angeordnet ist oder dass das Reaktionsflächenelement (3) wenigstens 50% der Innenoberfläche der Luftreinigungsvorrichtung (10) einnimmt,
**dadurch gekennzeichnet dass**
entweder als Reaktionsflächenelement (3) ein mit dem Katalysatormaterial als Oberflächenbeschichtung versehenes Drahtgeflecht vorgesehen ist oder das Reaktionsflächenelement (3) als oberflächenbeschichtetes Schüttgut (11) in Form von Glasrohrabschnitten und/oder in Form von Glaskugeln ausgebildet ist.

2. Luftreinigungsvorrichtung (10) nach Anspruch 1, **dadurch gekennzeichnet, dass** das Reaktionsflächenelement (3) in wenigstens 60% des Luftführungsvolumens angeordnet ist, bevorzugt in wenigstens 80% des Luftführungsvolumens.

3. Luftreinigungsvorrichtung (10) nach einem der vorhergehenden Ansprüche, **dadurch gekennzeichnet, dass** das Katalysatormaterial Titandioxid enthält.

4. Luftreinigungsvorrichtung (10) nach einem der vorhergehenden Ansprüche, **dadurch gekennzeichnet, dass** die Luftreinigungsvorrichtung (10) ein weiteres Luftführungsvolumen aufweist, wobei das weitere Luftführungsvolumen ein Zeolith-Material und/oder ein Silber-Substrat-Material als ein weiteres Katalysatormaterial aufweist.

5. Luftreinigungsvorrichtung (10) nach einem der vorhergehenden Ansprüche, **dadurch gekennzeichnet, dass** das Reaktionsflächenelement (3) ein Fasermaterial aufweist, bevorzugt ein Zeolith-Material und/oder ein Silber-Substrat-Material.

6. Luftreinigungsvorrichtung (10) nach einem der vorhergehenden Ansprüche, **dadurch gekennzeichnet, dass** die Luftreinigungsvorrichtung (10) ein Basiselement (21) und ein Deckelement (22) aufweist, wobei das Reaktionsflächenelement (3) mit dem Basiselement (21) oder dem Deckelement (22) einstückig verbunden ist, insbesondere in Form von in das Luftführungsvolumen hineinragenden oberflächenbeschichteten stabförmigen Elementen (23).

7. Luftreinigungsvorrichtung (10) nach einem der vorhergehenden Ansprüche, **dadurch gekennzeichnet, dass** das Luftführungsvolumen eine wenigstens zweifache Biegung um mehr als 90° zwischen Lufteinlass (1) und Luftauslass (2) aufweist.

8. Luftreinigungsvorrichtung (10) nach einem der vorhergehenden Ansprüche, **dadurch gekennzeichnet, dass** das Luftführungsvolumen zwischen Lufteinlass (1) und Luftauslass (2) spiralförmig ausgebildet ist.

9. Luftreinigungsvorrichtung (10) nach einem der vorhergehenden Ansprüche, **dadurch gekennzeichnet, dass** als Lichtquelle (4) eine Mehrzahl von Leuchtdioden vorgesehen sind.

10. Luftreinigungsvorrichtung (10) nach einem der vorhergehenden Ansprüche, **dadurch gekennzeichnet, dass** die Luftreinigungsvorrichtung (10) in eine Fahrzeugkarosserie (30), insbesondere den Dachbereich der Fahrzeugkarosserie (30), derart integriert ist, dass ein Teil der Fahrzeugkarosserie (30) als oberflächenbeschichtete Wandung (31) des Luftführungsvolumens vorgesehen ist.

11. Luftreinigungsvorrichtung (10) nach einem der Ansprüche 1 bis 9, **dadurch gekennzeichnet, dass** die Luftreinigungsvorrichtung (10) in einer Fahrzeugkomponente (40), insbesondere einem Dachhimmel, derart integriert ist, dass ein Teil der Fahrzeugkomponente (40) als oberflächenbeschichtete Wandung (41) des Luftführungsvolumens vorgesehen ist.

12. Fahrzeugkomponente (40) mit einer integrierten Luftreinigungsvorrichtung (10) gemäß Anspruch 1 wobei die Fahrzeugkomponente ein Dachhimmel oder eine Hutablage oder eine Mittelkonsole oder eine Türverkleidung oder A-Säulenverkleidung oder eine B-Säulenverkleidung oder eine C-Säulenverkleidung oder eine Instrumententafel ist, wobei die Luftreinigungsvorrichtung (10) in die Fahrzeugkomponente (40) derart integriert ist, dass ein Teil der Fahrzeugkomponente (40) als oberflächenbeschichtete Wandung (41) des Luftführungsvolumens vorgesehen ist.

13. Verwendung einer Fahrzeugkarosserie (30) oder eines Teils davon als Wandung einer Luftreinigungsvorrichtung (10) gemäß Anspruch 10.

14. Verwendung einer Fahrzeugkomponente (40) oder eines Teils davon als Wandung einer Luftreinigungsvorrichtung (10) gemäß Anspruch 11.

## Claims

1. Air purification device (10) for a vehicle, having an air inlet (1), an air outlet (2) and an air volume between the air guide inlet (1) and
Air outlet (2), wherein in the air flow volume of at least one reaction surface element (3) is provided, in the air flow volume at least one light source (4) is arranged for ultraviolet light, the surface of the reaction surface element (3) comprises a catalyst material and the reaction surface element (3) is essentially provided in the entire air flow volume or the reaction surface element (3) occupies at least 50% of the inner surface of the air purification device (10),
**characterised in that**
either the reaction surface element (3) is a wire mesh with the catalyst material as a surface coating, or the reaction surface element (3) is formed as a surface-coated bulk material (11) in the form of glass tube sections and/or in the form of glass globes.

2. Air purification device (10) according to claim 1, **characterised in that** the reaction surface element (3) is arranged to at least 60% of the air flow volume, preferably, however, in at least 80% of the air flow volume.

3. Air purification device (10) according to one of the preceding claims, **characterised in that** the catalyst material contains titanium dioxide.

4. Air purification device (10) according to one of the preceding claims, **characterised in that** the air purification device (10) has a further air flow volume, wherein the further air flow volume comprises a zeolite material and/or a silver substrate material as a further catalyst material.

5. Air purification device (10) according to one of the preceding claims, **characterised in that** the reaction surface element (3) comprises a fibre material, preferably a zeolitic material and/or a silver substrate material.

6. Air purification device (10) according to one of the preceding claims, **characterised in that** the air purification device (10) is integrally connected to a base element (21) and a base element (21) or the cover element (22), in particular in the form of the surface-coated rod-like elements (23) protruding into the air flow volume.

7. Air purification device (10) according to one of the preceding claims, **characterised in that** the air flow volume is provided in at least a two-fold deflection by more than 90° between the air inlet (1) and air outlet (2).

8. Air purification device (10) according to one of the preceding claims, **characterised in that** the air flow volume between the air inlet (1) and air outlet (2) is designed spirally.

9. Air purification device (10) according to one of the preceding claims, **characterised in that** a plurality of light-emitting diodes are provided as a light source (4).

10. Air purification device (10) according to one of the preceding claims, **characterised in that** the air purification device (10) is integrated into a vehicle body (30), in particular the roof of the vehicle body (30) so that a portion of the vehicle body (30) is provided as a surface-coated wall (31) of the air flow volume.

11. Air purification device (10) according to one of the claims 1 to 9, **characterised in that** the air purification device (10) is integrated in a vehicle component (40), in particular a roof lining, so that a part of the vehicle component (40) is provided as a surface-coated wall (41) of the air flow volume.

12. Vehicle component (40) according to claim 1, with an integrated air purification device (10), the vehicle component roof liner or a rear parcel shelf, or a central console or a door trim or an A-pillar trim panel or B-pillar trim or a C-pillar trim or an instrument panel, whereby the air purification device ( 10) is integrated into the vehicle component (40) so that a part of the vehicle component (40) is functioning as a surface-coated wall (41) of the air flow volume.

13. Vehicle body (30) according to Claim 10, or a part thereof, which is used as a wall of an air purification device (10)

14. Vehicle component (40) according to Claim 11, or a part thereof, which can be used as a wall of an air purification device (10).

## Revendications

1. Dispositif d'épuration d'air (10) pour véhicule, comportant une arrivée d'air (1), une sortie d'air (2) et un volume de conduction d'air entre l'arrivée d'air (1) et la sortie d'air (2), étant prévu dans le volume de conduction d'air au moins un élément à surface réactionnelle (3), au moins une source lumineuse (4) pour de la lumière ultraviolette étant disposée dans le volume de conduction d'air, la surface de l'élément à surface réactionnelle (3) contenant un matériau catalyseur et l'élément à surface réactionnelle (3) étant disposé substantiellement dans tout le volume de conduction d'air ou l'élément à surface réactionnelle (3) occupant au moins 50 % de la surface interne du dispositif d'épuration de l'air (10),
**caractérisé en ce que**
soit il est prévu comme élément à surface réactionnelle (3) un treillis métallique pourvu du matériau catalyseur faisant office de revêtement de surface, soit l'élément à surface réactionnelle (3) se présente sous la forme d'un produit en vrac (11) à surface revêtue prenant la forme de tronçons de tubes de verre et/ou de billes de verre.

2. Dispositif d'épuration d'air (10) selon la revendication 1, **caractérisé en ce que** l'élément à surface réactionnelle (3) est disposé dans au moins 60 % du volume de conduction d'air, de préférence dans au moins 80 % du volume de conduction d'air.

3. Dispositif d'épuration d'air (10) selon une des revendications précédentes, **caractérisé en ce que** le matériau catalyseur contient du dioxyde de titane.

4. Dispositif d'épuration d'air (10) selon une des revendications précédentes, **caractérisé en ce que** le dispositif d'épuration d'air (10) présente un autre volume de conduction d'air, l'autre volume de conduction d'air contenant un matériau à base de zéolithe et/ou un matériau à base de substrat d'argent en tant qu'autre matériau catalyseur.

5. Dispositif d'épuration d'air (10) selon une des revendications précédentes, **caractérisé en ce que** l'élément à surface réactionnelle (3) contient un matériau fibreux, de préférence un matériau à base de zéolithe et/ou un matériau à base de substrat d'argent.

6. Dispositif d'épuration d'air (10) selon une des revendications précédentes, **caractérisé en ce que** le dispositif d'épuration d'air (10) est un élément de base (21) ou forme bloc avec un élément de base (21) ou l'élément couvercle (22), en particulier sous forme d'éléments (23) prenant la forme de barres à surface revêtue saillant dans le volume de conduction d'air.

7. Dispositif d'épuration d'air (10) selon une des revendications précédentes, **caractérisé en ce que** le volume de conduction d'air présente au moins une double courbure de plus de 90° entre l'arrivée d'air (1) et la sortie d'air (2).

8. Dispositif d'épuration d'air (10) selon une des revendications précédentes, **caractérisé en ce que** le volume de conduction d'air est réalisé en forme spiralée entre l'arrivée d'air (1) et la sortie d'air (2).

9. Dispositif d'épuration d'air (10) selon une des revendications précédentes, **caractérisé en ce qu'**il est prévu comme source lumineuse (4) une pluralité de diodes électroluminescentes.

10. Dispositif d'épuration d'air (10) selon une des revendications précédentes, **caractérisé en ce que** le dispositif d'épuration d'air (10) est intégré dans une carrosserie de véhicule (30), en particulier la partie toit de la carrosserie de véhicule (30), de manière à ce qu'une partie de la carrosserie de véhicule (30) se présente sous forme d'une paroi à surface revêtue (31) du volume de conduction d'air.

11. Dispositif d'épuration d'air (10) selon une des revendications 1 à 9, **caractérisé en ce que** le dispositif d'épuration d'air (10) est intégré dans un composant de véhicule (40), en particulier un ciel de toit, de manière à ce qu'une partie du composant de véhicule (40) se présente sous forme d'une paroi à surface revêtue (31) du volume de conduction d'air.

12. Composant de véhicule (40) à dispositif d'épuration d'air (10) selon la revendication 1, le composant de véhicule étant un ciel de toit ou une lunette arrière ou une console centrale ou un habillage de portière ou un habillage de colonne A ou un habillage de colonne B ou un habillage de colonne C ou un tableau de bord, le dispositif d'épuration d'air (10) étant intégré dans le composant de véhicule (40) de manière à ce qu'une partie du composant de véhicule (40) se présente sous forme d'une paroi à surface revêtue (41) du volume de conduction d'air.

13. Utilisation d'une carrosserie de véhicule (30) ou d'une partie de celle-ci comme paroi d'un dispositif d'épuration d'air (10) selon la revendication 10.

14. Utilisation d'une composant de véhicule (40) ou d'une partie de celle-ci comme paroi d'un dispositif d'épuration d'air (10) selon la revendication 11.
